# EUROPEAN PATENT SPECIFICATION

(11) **EP 2 527 002 B1**
(45) Date of publication and mention of the grant of the patent: **04.03.2020**
(21) Application number: 11732926.8
(22) Date of filing: 13.01.2011
(51) Int. Cl.: A61N 1/44, H01T 23/00

(54) **METHOD FOR INCREASING MOISTURE CONTENT OF SKIN SURFACE AND IMPROVING MOISTURE-RETAINING FUNCTION OF DERMIS AND BEAUTY DEVICE THEREFOR**
VERFAHREN ZUR ERHÖHUNG DES FEUCHTIGKEITSGEHALTS DER HAUTOBERFLÄCHE UND ZUR VERBESSERUNG DER FEUCHTIGKEITSERHALTUNGSFUNKTION DER HAUT SOWIE KOSMETIKVORRICHTUNG DAFÜR
PROCÉDÉ D'AUGMENTATION DE TENEUR EN HUMIDITÉ DE SURFACE DE LA PEAU ET D'AMÉLIORATION DE FONCTION DE RETENUE D'HUMIDITÉ DU DERME ET DISPOSITIF DE PRODUIT DE BEAUTÉ ASSOCIÉ

(30) Priority: 18.01.2010 JP 2010007946
(43) Date of publication of application: 28.11.2012
(73) Proprietor: Sharp Kabushiki Kaisha, Sakai City, Osaka 590-8522 (JP)
(72) Inventor: NISHIUCHI, Emi, Sakai City, Osaka 590-8522 (JP); OKANO, Hiroaki, Sakai City, Osaka 590-8522 (JP); MORIMOTO, Katsushi, Sakai City, Osaka 590-8522 (JP); MATSUOKA, Norihiro, Sakai City, Osaka 590-8522 (JP); NISHIKAWA, Kazuo, Sakai City, Osaka 590-8522 (JP); SAKIKAWA, Nobuki, Sakai City, Osaka 590-8522 (JP)
(74) Representative: Treeby, Philip David William
(86) International application number: PCT/JP2011/050439
(87) International publication number: WO 2011/087056

(56) References cited:
- EP-A1- 1 637 811
- EP-A2- 1 285 599
- JP-A- 2002 216 933
- JP-A- 2003 185 167
- JP-A- 2005 083 651
- JP-A- 2005 224 352
- JP-A- 2005 224 352
- US-A1- 2006 285 269
- US-A1- 2008 097 279
- US-B1- 6 632 407

## Description

### [Technical Field]

The present invention relates to a method for increasing a moisture content of a skin and skin elasticity by ions, and a facial care apparatus for increasing the moisture content of a skin and skin elasticity.

### [Background Art]

Conventionally, in order to increase the moisture content of a skin and improve skin elasticity, the method of coating the skin with a coating agent containing a moisturizing component has been generally carried out. Besides, Patent Literature 1 discloses the method which suppresses drying of a skin and increasing the moisture content of the skin by increasing the moisture content of the environment by spraying moisture into the air by a humidifying device. Further, Patent Literature 2 discloses the method which condenses the moisture in the air, atomizes the condensed moisture and sprays it, but this method needs to provide the function of making the temperature of the air low for condensation of the moisture and causing dew condensation and flocculation, and needs to use an expensive element such as a Peltier element. Further, there have been the problems that the power consumption increases and the device is upsized at the same time.

Further, an electric field is further applied to generated steam to change the steam to charged water droplets called "steam ions", and the water droplets are supplied. (For example, Patent Literature 3)

Further, recently, water droplets of a nano size are made by using an ultrasonic element, an electric field is further applied to the water droplets, and the charged water droplets in the form of ultra-fine particles are made and supplied. (For example, Patent Literature 4)

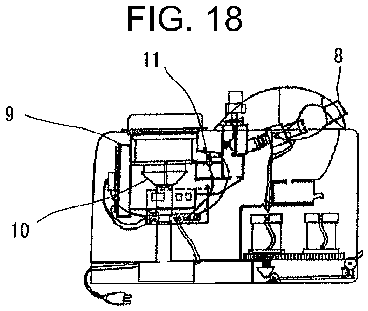

Explaining the device according to FIG. 18, water for making steam is supplied to a boiler 10, and is warmed by a heater 9 to generate steam instantly. Subsequently, the steam is subjected to corona discharge with an ion steam generator 11 to contain ions, and is injected to a face from an ejection port 8.

However, if the water in the tank which is supplied to generate steam, mist and the like becomes insufficient, water has to be added thereto, and much effort is required.

Further, the water in the supply water tank is left for a long time, whereby saprophytic bacteria increase, and the water may be in an unhygienic state. If Legionella pneumophila and the like have grown, a trouble is likely to occur to the users, and as a result, usability is reduced.

JP 2005-224352 discusses a method for giving advice on beauty treatment and an ion measuring apparatus. US 6632407 B1 discloses a personal electro-kinetic air transporter-conditioner involving electro-kinetic conversion of electrical energy into fluid flow of an ionizable dielectric medium in which an electro-kinetically produced flow of air is created. EP 1637811 A1 discloses an ion generator and an air conditioning apparatus for sterilizing air in a room.

US 2008/097279 A1 discusses a cosmetic apparatus for eliminating the generation of harmful substances to a skin.

### [Citation List]

### [Patent Literature]

[Patent Literature 1] Japanese Patent Laid-Open Publication No. 2009-78245
[Patent Literature 2] Japanese Patent Laid-Open Publication No. 2006-61407
[Patent Literature 3] Japanese Patent Laid-Open Publication No. 2007-75243
[Patent Literature 4] Japanese Patent Laid-Open Publication No. 2007-296284

JP 2005-224352

US 6,632,407

### [Summary of Invention]

### [Technical Problem]

The present invention is invented in view of the above described conventional problems, and provides a beauty apparatus which does not need to generate steam, mist and the like.

### [Solution to Problem]

The outline of the solution to the problem according to the present invention will be described hereinafter.

In accordance with the present invention, there are provided a method for increasing a moisture content of a skin surface and improving a moisture retaining function of a dermis by a beauty apparatus as recited by claim 1, and a beauty apparatus for increasing moisture content of a skin surface and improving a moisture retaining function of a dermis as recited by claim 3.

Preferred embodiments are defined by the depended claims.

Aspects, embodiments, examples or methods of the present disclosure which do not fall within the scope of the appended claims do not form part of the present invention.

According to the present disclosure there is disclosed a method for increasing a moisture content of a skin surface and improving a moisture retaining function of a dermis, characterized in that: irradiating a skin surface simultaneously with positive ions and negative ions which are generated from an air by an electric discharge in an atmosphere.

The present method brings about the effect of increasing a skin moisture content and improving skin elasticity by the ions generated by an electric discharge and does not perform humidification using water, and therefore, the humidity in a room does not rise. Further, the moisture in the air is not condensed, and therefore, the advantages are provided, that the device cost does not significantly increase, the power consumption is small and the device is not upsized.

The method for increasing a moisture content in a human skin surface and improving skin elasticity is characterized in that concentrations of the positive and negative ions are each 7000/cm³ or more.

As a result of performing a study by changing the concentrations of the positive and negative ions, it has become obvious that the effect of increasing the skin moisture content and the effect of improving skin elasticity can be obtained by generating ions of 7000/cm³ or more.

Preferably, the method for increasing a moisture content in a human skin surface and improving skin elasticity of the present invention uses H₃O⁺(H₂O)m (m = a natural number such as 0, 1, 2, 3 ...) as the positive ions, and O₂⁻(H₂O)n (n = a natural number such as 0, 1, 2, 3 ...) as the negative ions.

The mechanism of the effect of increasing the skin moisture content by both the positive and negative ions is not clear, but it is conceivable that
1) By simultaneous irradiation with H+ ions and O₂⁻ ions, -OH groups adhere to a skin surface, the skin surface is locally hydrophilized, and water molecules adhere and easily penetrates into the skin.
2) Water molecules surrounding the ions are taken into the skin, and the like.

The water molecules surrounding the ions are due to the water molecules which are originally present in the air, and the amount of the water molecules which are in contact with a skin is not significantly increased. Accordingly, it is conceivable that a large skin moisture increasing effect cannot be obtained by 2), and it is obvious that 1) brings about a large effect.

From the present mechanism, it is important to contain H⁺ and O₂⁻ as ions, and it is estimated that thereby, OH groups are generated, and the effect of increasing the skin moisture is brought about.

Meanwhile, skin elasticity generally depends on the composition of a deeper portion (dermis) of a skin. Improvement of the skin elasticity is considered to be the result that the moisture of the skin surface increases to thereby increase the content of the moisture kept in the dermis, and the elasticity of the skin is improved. More specifically, the skin elasticity improving effect is estimated as being obtained as the secondary action of the effect of increasing the skin moisture content by ions.

According to a second aspect of the present invention there is provided a beauty apparatus (100) for increasing moisture content of a skin surface and improving a moisture retaining function of a dermis, as defined in claim 3.

Preferably, the beauty apparatus for increasing a moisture content of a skin surface and improving a moisture retaining function of a dermis, wherein as for the ions for irradiating the skin, H₃O⁺(H₂O)m (m is 0 or an optional natural number) as the positive ions, and O₂⁻ (H₂O)n (n is 0 or an optional natural number) as the negative ions are used.

The beauty apparatus of the present invention irradiates a user with the positive and negative ions which are generated by using the ion generation element by carrying the positive and negative ions by an air flow at a low speed, and increases the concentration of the ions for irradiating the skin surface to a predetermined value or more, whereby the positive and negative ions react to produce water, which can moisten the skin and can improve the elasticity of the skin. Further, the blowoff port ion detector is included so that the ion concentration becomes high when the ions which are supplied from the ion generation means are released from the blowoff port, and thereby the blowoff velocity is controlled to be kept at a low level while the ion concentration is elevated.

### [Advantageous Effect of Invention]

The method for increasing the moisture content in a human skin surface and improving the moisture retaining function of a dermis, and the beauty apparatus according to the present invention bring about the effect of increasing the skin moisture content and the effect of improving the skin elasticity by irradiating the skin of a user with the positive and negative ions which are generated by an electric discharge, and because the method and apparatus do not perform humidification using water, the method and apparatus do not require the storage, replenishment and sanitary supervision of water, or do not increase the humidity in the room. Further, the method and the apparatus do not use water, and therefore, the beauty apparatus which can be easily used at any time can be provided. Further, the skin can be prevented from being dried by controlling the wind velocity, and the amount of water generated on the skin surface can be increased by controlling the ion concentration.

Furthermore, a water storage tank and a heating device for condensing the moisture in the air are not required, and therefore, the advantages are provided, that the entire device can be made compact, the device is easily handled and portable, the device cost does not significantly increase, the power consumption is small, and the device is not upsized.

In order that the present invention be more readily understood, specific embodiments thereof will now be described with reference to the corresponding drawings.

### [Brief Description of Drawings]

[FIG. 1] FIG. 1 is a schematic view showing the principle of an ion generation element.
[FIG. 2] FIG. 2 is a schematic view showing an ion generation apparatus and a blower fan.
[FIG. 3] FIG. 3 is a graph of change in skin moisture content according to embodiment 1.
[FIG. 4] FIG. 4 is a graph of change in skin moisture content according to embodiment 2.
[FIG. 5] FIG. 5 is a graph of change in skin moisture content according to comparative example 1.
[FIG. 6] FIG. 6 is a graph of change in skin moisture content according to comparative example 2.
[FIG. 7] FIG. 7 is a graph of change in skin moisture content according to embodiment 2.
[FIG. 8A] FIG. 8A is a graph of change in skin elasticity R5 according to embodiment 2.
[FIG. 8B] FIG. 8B is a graph of change in skin elasticity R7 according to embodiment 2.
[FIG. 9] FIG. 9 is a conceptual view showing a state of use of a facial care apparatus of the present invention.
[FIG. 10] FIG. 10 is a conceptual view of a structure of the facial care apparatus of the present invention.
[FIG. 11] FIG. 11 is a conceptual view of a wind directing body of the present invention, (a) is a plan view, (b) is a sectional view taken along the line A-A, and (c) is a sectional view taken along the line B-B.
[FIG. 12] FIG. 12 is a conceptual view showing the flow of ions of the present invention.
[FIG. 13] FIG. 13 is an external view of an ion generation device, (a) is a top view, (b) is a plan view and (c) is a right side view.
[FIG. 14] FIG. 14 is an example of a circuit of the ion generation device.
[FIG. 15] FIG. 15 is a circuit diagram of a wind velocity detector.
[FIG. 16] FIG. 16 is a control block diagram of the facial care apparatus.
[FIG. 17] FIG. 17 is a circuit diagram of an ion detector.
[FIG. 18] FIG. 18 is a structural view of a conventional facial care apparatus.

### [Description of Embodiments]

A mode for carrying out the present invention will be described in detail with reference to the drawings.

### [Embodiment 1]

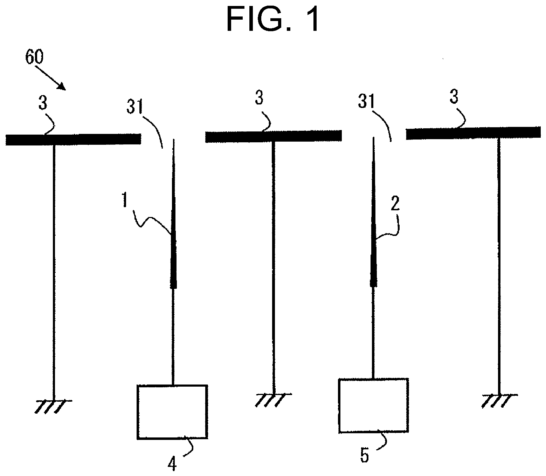

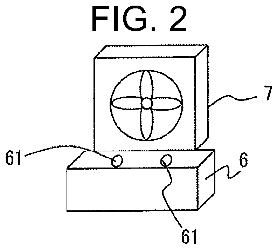

FIG. 1 is a schematic view explaining the principle of an ion generation element according to the present invention, and FIG. 2 is a conceptual view of an ion generation apparatus using the ion generation element.

An ion generation apparatus 6 houses an ion generation element 60 shown in FIG. 1 inside a case. The ion generation element 60 has voltage application needle electrodes 1 and 2 connected to high-voltage generation devices 4 and 5, and ground electrodes 3 disposed adjacently to the aforesaid voltage application needle electrodes. In the ground electrodes 3, the same number of through-holes 31, which allow the voltage application needle electrodes 1 and 2 to penetrate therethrough, as the number of voltages application needle electrodes are placed. The voltage application needle electrodes 1 and 2 have needle-shaped tip ends, and are disposed so that the voltage application needle electrodes 1 and 2 are located in centers of through-holes 31 of the ground electrodes 3 with the voltage application needle electrodes 1 and 2 connected to and supported by the high-voltage generation devices 4 and 5, and the needle-shaped tip ends are each located within the range of the thickness of the through-hole 31. The high-voltage generation devices 4 and 5 supply DC pulse voltages to the voltage application needle electrodes 1 and 2 to cause an electric discharge to ionize the air in the vicinity of the ground electrodes 3. Ions generated from the ion generation element 60 of the ion generation apparatus 6 are released from an opening 61 of the case. Subsequently, the released ions are diffused into the atmosphere by a blowing fan 7 which is installed adjacently to the ion generation apparatus 6.

The principle of ion generation of the ion generation element 60 will be described.

As the ion generation electrodes of the ion generation element 60, the voltage application needle electrode 1 (positive ion generation electrode) connected to the high-voltage generation device 4, and the voltage application needle electrode 2 (negative ion generation electrode) connected to the high-voltage generation device 5 are included. A DC pulse voltage is supplied to the aforesaid voltage application needle electrode 1, and a positive voltage is applied. A DC pulse voltage is supplied to the voltage application needle electrode 2, and a negative voltage is applied. By a corona discharge, the air in the vicinity of the positive ion generation electrode 1 and the ground electrode 3 is positively ionized, and the air in the vicinity of the negative ion generation electrode 2 and the ground electrode 3 is negatively ionized.

The amount (concentration) of the ions which are generated is regulated according to the voltage/pulse period, which is applied to the voltage application needle electrodes 1 and 2 by the high-voltage generation devices 4 and 5.

Next, experiments were conducted on change of the moisture content of a skin by air containing the positive ions and negative ions generated by the ion generation element 60 and the air which does not contain the positive ions and negative ions.

### <Experiment method>

A partition plate was placed in the center of a face which was a test object, and the face was divided into a left face and a right face. The positive and negative ions which were generated from the ion generation element were fed (irradiated) to a left cheek of the face by the fan 7, and only the air which did not contain ions was blown (irradiated) to a right cheek. In this case, the distance between the ion generation element and the face was set at 30 cm. Further, the wind velocity at the face was set at 1 m/sec. The skin moisture content was measured with WSK-P500U (made by WaveCyber Corp. trade name).

As a result of analyzing the structures of the ions generated from the present ion generation electrodes with a TOF-mass spectrometer (time-of-flight mass spectrometer), generation of H₃O⁺(H₂O)m (m = a natural number such as 0, 1, 2, 3 ...) as positive ions and O₂⁻ (H₂O)n (n = a natural number such as 0, 1, 2, 3 ...) as negative ions was able to be confirmed.

### Experiment 1

The voltages applied to the positive and negative ion generation electrodes (high-voltage application needle electrodes 1 and 2) of the ion generation element by the high-voltage generation devices 4 and 5 and the pulse periods were regulated so that the ion concentration on the face became 7,000 /cm³.

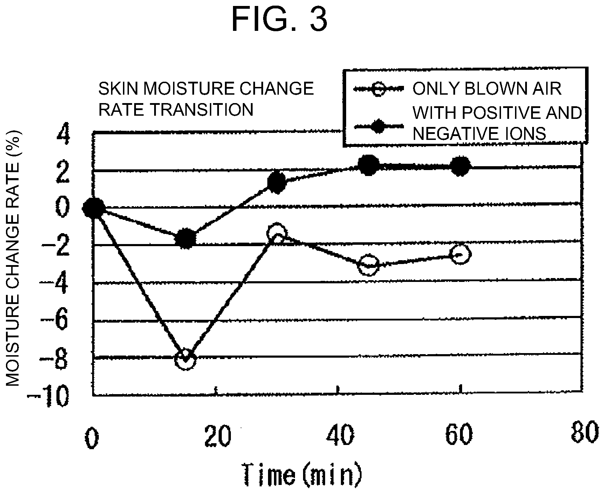

The result of measuring the change of the moisture content of the surface (skin) in this manner is shown in FIG. 3. The graph shown in FIG. 3 shows the change over time of the moisture contents of the face (skin) to which the air containing positive and negative ions are applied (irradiated), and the face (skin) to which only the air containing no ions is applied (irradiated). According to the graph, the result that the face (skin) irradiated with positive and negative ions increased in moisture content of the face (skin) with irradiation time as compared with the face (skin) irradiated with only blown air was obtained.

### Experiment 2

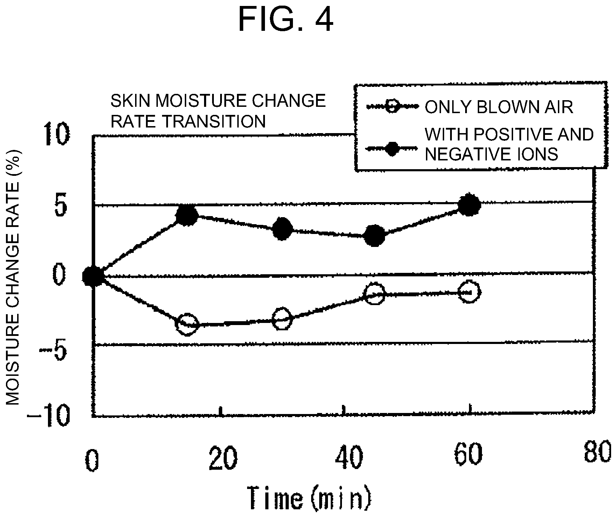

With the same method as in experiment 1, the change of the moisture content of the face (skin) was measured. However, the voltages of the high-voltage generation devices 4 and 5 which were applied to the ion generation electrodes (high-voltage application needle electrodes 1 and 2) and the pulse periods were regulated so that the concentration of the ions for irradiation was 25000/cm³. The result of measuring the change of the moisture content of the face (skin) is shown in FIG. 4. According to the graph, the result that the face (skin) irradiated with the positive and negative ions increased in the moisture content of the face (skin) with the irradiated time as compared with the face (skin) irradiated with only the blown air was obtained.

### Comparative example 1

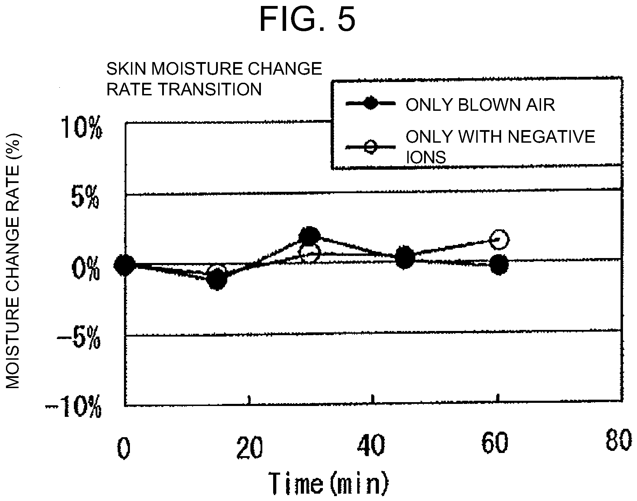

The change of the moisture content of the face (skin) was measured by the same method as in example 1. However, the irradiated ions were only negative ions, and the ion concentration was regulated to 7000/cm³. The result of measuring the change of the moisture content of the face (skin) is shown in FIG. 5. According to the result, in the case of irradiation of only the negative ions, the effect of increasing the skin moisture content was not obtained as compared with the face (skin) irradiated with only blown air.

### Comparative example 2

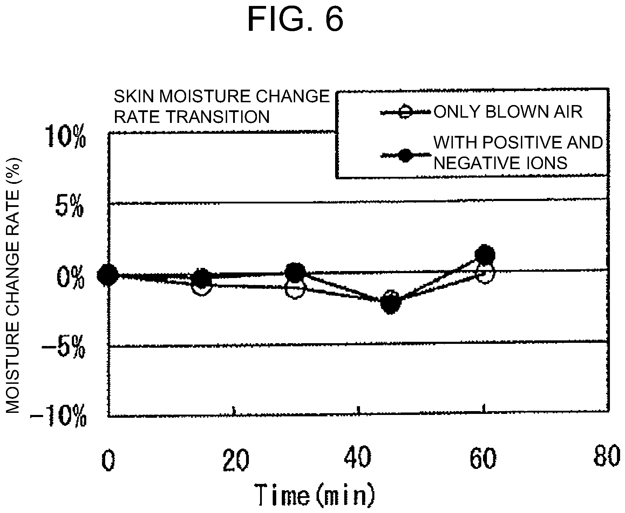

With the same method as in experiment 1, the change of the moisture content of the face (skin) was measured. However, the voltages which the high-voltage generation devices 4 and 5 applied to the ion generation electrodes (high-voltage application needle electrodes 1 and 2) and the pulse periods were regulated so that the concentration of the ions to be irradiated was 3000/cm³. The result of measuring the change of the moisture content of the face (skin) is shown in FIG. 6. According to the result, by irradiation with the ion concentration of 3000/cm³, the effect of increasing the skin moisture content was not obtained as compared with the face (skin) irradiated with only blown air.

From the above described experiments, it has been found out that the moisture contents of the faces (skins) supplied with the positive ions and the negative ions having the compositions of the positive ions H₃O⁺ (m is 0 or an optional natural number) and the negative ions O₂⁻ (H₂O)n (n is 0 or an optional natural number) with the ion concentrations set at 7000/cm³ to 25000/cm³ increase over time.

The reason why the ions are adsorbed by the face (skin) as above is considered to be because the nano-size water molecules which are generated by the positive ions and the negative ions having the compositions of the positive ions H₃O⁺(H₂O)m (m is 0 or an optional natural number) and the negative ions O₂⁻(H₂O)n (n is 0 or an optional natural number) which are supplied from the ion generation element being intensively hit against the skin, and are expressed by the following chemical formulas are effectively adsorbed by the skin.

(1) H₃O⁺ + O₂⁻ → ·OH + H₂O₂

(2) H₃O⁺ + O₂⁻ → ·O₂H + H₂O

(3) 2H₂O₂ → 2H₂O + O₂

More specifically, it is estimated that by applying H⁺ ions and O₂⁻ ions to the skin surface at the same time, -OH groups adhere to the skin surface, the skin surface is locally hydrophilized, water molecules easily penetrate through the skin, and bring about the effect of increasing the skin moisture.

As shown in the above embodiment, the positive and negative ions which are generated by an electric discharge increase the moisture contents in the human skin surfaces. Since the method increases the skin moisture content by positive and negative ions and does not use water, there is no increase of saprophytic bacteria, and since humidification is not performed, the humidity in a room is not elevated. Further, there is no need to condense moisture in the air, and therefore, the advantages that the device cost is not increased significantly, the power consumption is small, and the device is not upsized are provided.

### [Embodiment 2]

Next, the experiment was conducted on change of the moisture content of a skin and skin elasticity by the air containing the positive ions and the negative ions generated by the ion generation element 60 and the air containing no such ions.

### <Experiment method>

The test was a single blind test randomization 2 plots cross-over test. Four days that are 3 days for the previous observation term and 1 day as the inspection day were set, and the test was carried out by dividing 16 women with normal and healthy bodies who were apt to feel drying of their skins into groups each with four people. The test plots were as follows.
Test plot 1: ion concentration 25,000/cm³ (25,000 group)
Test plot 2: no ion (control group)

The temperature of the test room was regulated by an air-conditioner with 28°C as the target. Further, the humidity was regulated by a desiccant type dehumidifier with 30 to 40% as the target.

The test subjects washed their faces with a cleansing milk and a facial cleansing foam, and after 60 minutes from the face washing, the test subjects were allowed to enter the test room. The test subjects were allowed to spend 140 minutes in total that are 20 minutes before operation of the test apparatus, 60 minutes after operation of the test apparatus, and 60 minutes after stoppage thereof, in the state in which the test subjects lay face up on their beds. This was carried out once in the morning and once in the afternoon. As for clothing on the inspection day, the test subjects wore the same T-shirts and sweat suits. The test subjects are instructed not to change their daily lives such as their eating habits and exercises up to then, and their daily lives were grasped by making them record their living situations such as sleeping hours, working hours, contents of meals, alcohol intake amounts, the use situation of medicines, physical conditions and the like in the day books everyday for 3 days before the inspection day. In consideration of the influence by meals, the same packed meals were taken by 21 o'clock as the dinner on the day before the inspection day, and intake of alcohol was prohibited. On the inspection day, the same meals were taken 1 hour before each of the inspections in the morning and the afternoon.

### (a) Skin moisture content

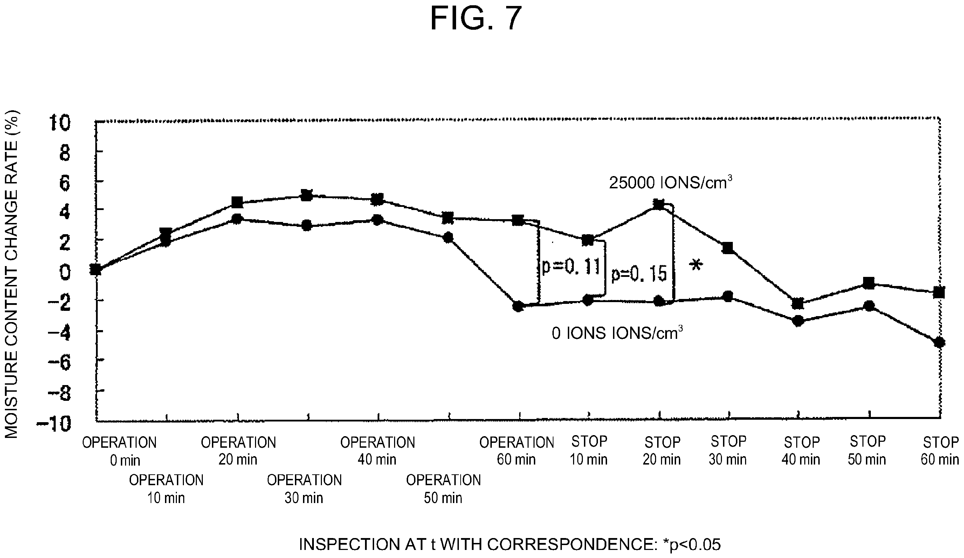

0 minutes, 10 minutes, 20 minutes, 30 minutes, 40 minutes, 50 minutes and 60 minutes after operating the ion generation device, and 10 minutes, 20 minutes, 30 minutes, 40 minutes, 50 minutes and 60 minutes after stopping it, the skin moisture contents at the same spots at 1 cm sideway from the left eyes were measured by using Corrneometer CM825 (Integral Corporation, trade name). The result of measuring the change of the moisture contents of faces (skins) in this manner is shown in FIG. 7. The graph shown in FIG. 7 shows the change over time of the moisture contents of the face irradiated with positive and negative ions (skin: shown by the black squares) and the face which were not irradiated with the positive and negative ions (skin: shown by the black circles).

According to the graph, the result was obtained, that the moisture contents of the faces (skins: shown by the black squares) irradiated with the positive and negative ions are continuously higher during irradiation with the positive and negative ions and after stoppage of the irradiation as compared with the faces (skins: shown by the black circles) not irradiated therewith.

### (b) Skin elasticity

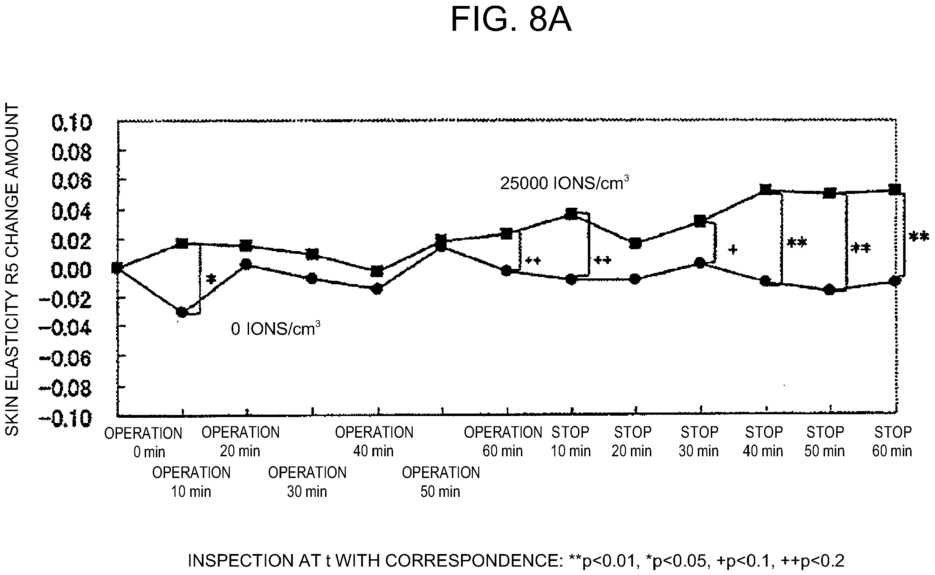

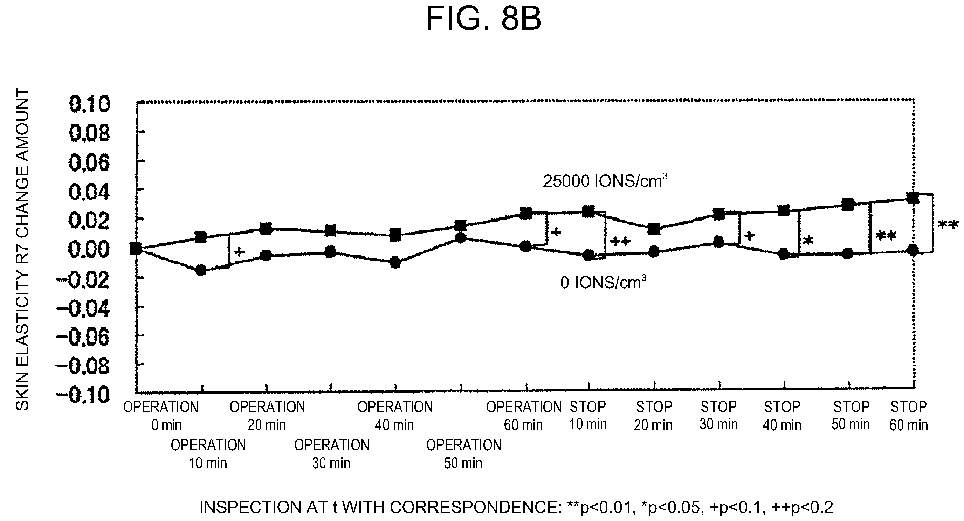

0 minute, 10 minutes, 20 minutes, 30 minutes, 40 minutes, 50 minutes and 60 minutes after operating the ion generation device, and 10 minutes, 20 minutes, 30 minutes, 40 minutes, 50 minutes and 60 minutes after stoppage thereof, the skin elasticity R5 (the restoration width for 0.1 seconds after starting release with respect to the suction width for 0.1 seconds after starting suction) and the skin elasticity R7 (the restoration width for 0.1 seconds after starting release with respect to total suction width for two seconds) at the same spots 1 cm under the left eyes were measured by using Cutometer MPA580 (Integral Corporation, trade name). The result of measuring the change of the skin elasticity in this manner is shown in FIGS. 8A and 8B. The graphs shown in FIGS. 8A and 8B show the change over time of the skin elasticity of the face (skin) irradiated with positive and negative ions and the skin elasticity of the face (skin) which were not irradiated therewith.

According to the graphs, the result was obtained, that in the faces (skins: shown by the black squares) of the test plot 1 irradiated with the positive and negative ions, the skin elasticity was continuously improved during irradiation with the positive and negative ions and after stopping the irradiation as compared with the faces (skin: shown by the black circles) of the test plot 2 not irradiated therewith. The effect of the skin elasticity appeared slightly later as compared with that the increase in the skin moisture content appeared immediately after the irradiation, and therefore, improvement in the skin elasticity is estimated as the secondary effect by the increase of the skin moisture content.

By the above described experiment, the moisture content of the faces (skins) of the test plot 1 supplied with ions with the ion concentrations of the positive ions and the negative ions having the compositions of the positive ions H₃O⁺(H₂O)m (m is 0 or an optional natural number) and the negative ions O₂⁻(H₂O)n (n is 0 or an optional natural number) set as 25,000/cm³ increases over time, and the skin elasticity (moisture retaining function of dermis) is improved therewith.

### [Embodiment 3]

Next, a beauty apparatus using the above described ion generation element will be described.

A mode for carrying out the beauty apparatus will be described by using the drawing. In an embodiment shown in the drawing, the case of a facial care apparatus as a beauty apparatus will be described.

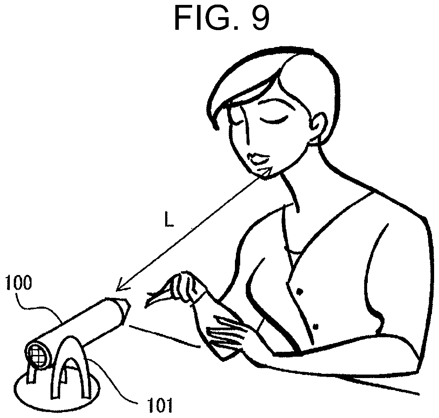

FIG. 9 is an explanatory view of a use state of a facial care apparatus (hereinafter, a facial care apparatus) 100 as a beauty apparatus. The facial care apparatus 100 is installed by being supported by a supporting member 101. The facial care apparatus 100 generates positive ions and negative ions, and is used in such a manner as to irradiate the face of a user with the ions which are released.

A structure of the facial care apparatus 100 will be described.

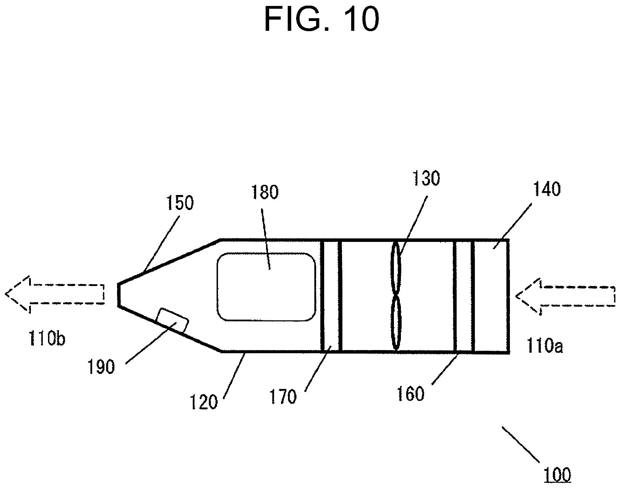

As shown in FIG. 10, the facial care apparatus 100 has a filter 160, a blower 130, a wind directing body 170, an ion generation device 180, a wind velocity detector 190 and the like mounted inside a casing 120. Further, switches (not illustrated) which instruct operation of the devices such as the blower 130, the ion generation device 180 and the wind velocity detector 190, a control unit which controls drive of the blower 130, the wind velocity detector 190, the ion generation device 180 and the like, an operation display device (not illustrated) and the like are arranged inside the casing 120.

When the blower 130 is operated, inflow air 110a flows into the casing 120 from an inlet port 140, passes through the wind directing body 170, and includes ions generated from the ion generator 180, and the air containing the ions is released from a release port 150 as released air 110b.

Because the blower 130 and the ion generation device 180 are housed in a wind tunnel inside the casing 120 of the facial care apparatus 100, the diameter of the casing 120 cannot be configured to be small. Accordingly, in order to generate ions and transfer/release the ions more efficiently by the air flowing in, the wind directing body 170 as will be described later is inserted into and placed inside the casing 120.

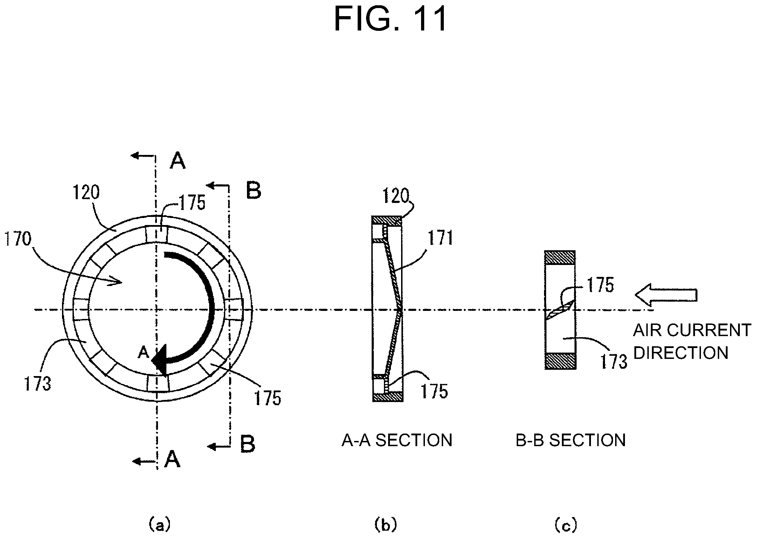

A configuration of the wind directing body 170 will be described according to FIG. 11.

The wind directing body 170 is installed at a downstream side of the blower 130. The wind directing body 170 is formed into a disk shape which is fitted in the casing 120, and a disk-shaped closure plate 171 which closes air which flows down is placed at a center portion. In a gap 173 between the closure plate 171 and the casing 120, blade pieces 175 for turning the air which flows down are placed at a plurality of spots. The blade pieces 175 are installed to be inclined to change the flow of the passing air to turn it. In the embodiment, eight of the blade pieces 175 are disposed at the peripheral edge of the closure plate 171 and around the center axis of the casing 120. The air which flows in by the blower 130 hits against the closure plate 170, and the air current which has its way closed spouts to the downstream side from the gap 173 at the peripheral portion and flows down. At this time, the downflow direction of the air current changes along the inclination of the blade pieces 175 and the air current is turned. This embodiment has the configuration in which by the eight blade pieces 175, the downflow air turns clockwise on the plan view shown by the direction A as shown in FIG. 11.

The ion generator 180 is disposed at the downstream side of the wind directing body 170.

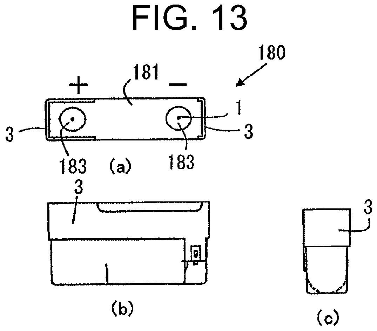

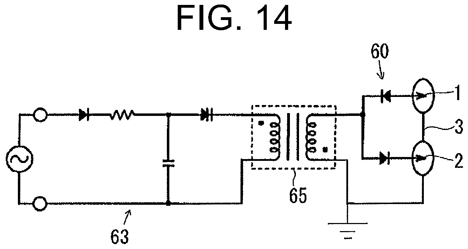

FIG. 13 is an external view of the ion generation device. A voltage application needle electrode 1 which is connected to a high-voltage generation device and a ground electrode 3 are included, openings 183 for releasing the positive ions and negative ions generated from the ion generation element to outside are bored in a lid 181. FIG. 14 is one example of a circuit of the ion generation device. The example shows a mode in which a drive circuit 63 and an ion generation unit 60 of the device are connected via a transformer 65. In the embodiment, the illustrated ion generation device is exemplified, but as the ion generation device, other ion generation devices also can be used as long as the ion generation devices can generate the aforementioned positive and negative ions.

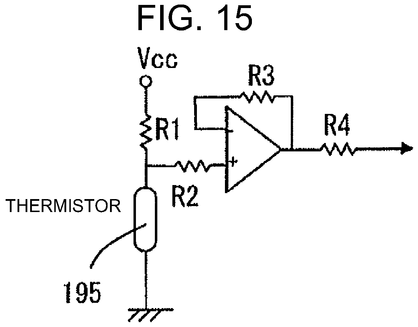

The wind velocity detector 190 is mounted in the vicinity of the blowoff port 150 at a downstream side of the ion generation device 180. FIG. 15 shows an example of a circuit of the wind velocity detector. The wind velocity detector 190 keeps a thermistor 195 self-heating, and the temperature of the thermistor 195 is reduced when an air current passes the thermistor 195. The wind velocity is detected by the temperature reduction. For detection of a wind velocity, there are a hot wire type and a thermistor type. Any type of the conventional wind velocity detectors can be used besides the illustrated type, and a similar effect is provided.

The number of ions which can be released from the ion generation device 180 is significantly influenced by the velocity of the wind which passes through the vicinity of the discharge electrodes. More specifically, the velocity of the wind in the vicinity of the electrodes is higher, the number of the ions which are released increases more. Accordingly, the wind velocity needs to be increased as much as possible in the vicinity of the ion generation device 180. Meanwhile, the upper limit of the velocity of the wind which is fed from the blowoff port 150 is approximately 1 m at the utmost to suppress drying of a face (skin). Thus, the present facial care apparatus has the wind velocity detector 190 installed at the blowoff port 150, detects the wind velocity in the vicinity of the blowoff port 150, and controls the velocity of the wind to be blown off.

The quantity of the air passing through the passage gap 173 (wind directing body 170) and the quantity of the air which is fed from the blowoff port 150 are basically the same. Therefore, the wind velocity in the vicinity of the ion generation device 180 is increased by narrowing the passage gap 173, and the sectional area of the blowoff port 150 is made larger than the passage gap 173, whereby the velocity of the wind which is fed from the blowoff port 150 can be made low. The air is compressed by the blowoff port 150 where the diameter of the casing is made smaller than the other casing diameter. The air current blown from the blowoff port temporarily causes contraction in the vicinity of the blowoff port 150, but thereafter, tends to be expanded. However, if the blowoff (release) wind velocity is made too low, the air current does not directly reach the object (face). In order to cause the air current to reach a distant place, it is effective not to expand the air current, and therefore, expansion of the air current which is blown off is suppressed by turning the downflow air current by the wind directing body 170.

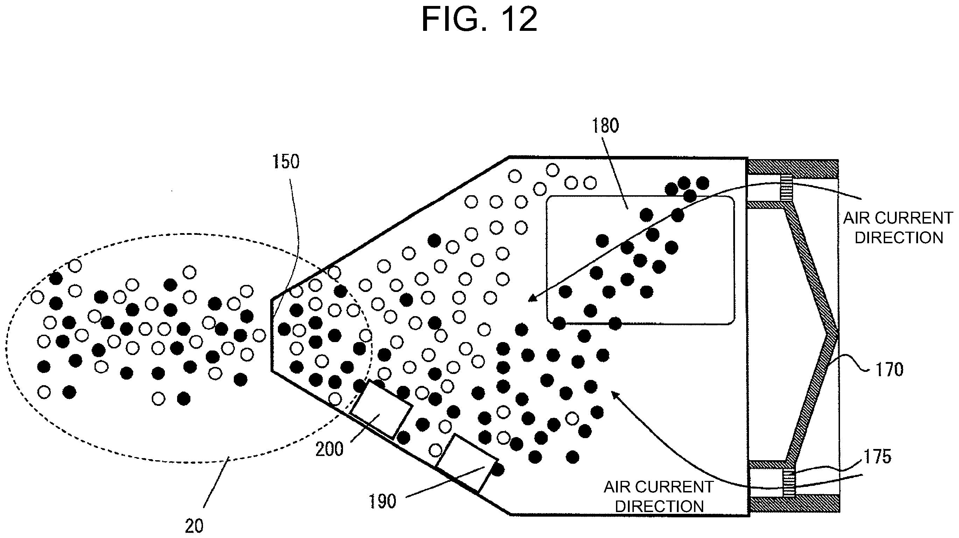

FIG. 12 schematically shows the behavior of the ions in the vicinity of the blowoff port.

The air which is enhanced in the downflow velocity with the downflow direction changed by the blade pieces 175 includes the ions (positive and negative ions) generated from the ion generation device 180, and the air containing the positive and negative ions (white circles and black circles) with high density is released from the blowoff port 150 to an ion compressed region 20 as illustrated. At this time, the port diameter of the blowoff port 150 is formed to be larger than the passage gap 173, whereby the release velocity is reduced. Further, the air which is blown from the blowoff port 150 changes (turns) the downflow direction by the blade pieces 175, and therefore, is configured to reach a face in the state with high-density ions without being expanded.

As above, in the facial care apparatus 100, the inflow air 110a taken in from the inlet port 140 by rotation of the fan 130 installed at the upstream side of the inside of the casing 120 is released as the released air 110b from the blowoff port 150 at the most downstream side. The air which flows in from the inlet port 140 during this while passes through the filter 160, has the flow direction changed by the wind directing body 170, includes the ions generated from the ion generation device 180 and is released to the external air from the blowoff port 150.

An operation of the facial care apparatus of the above described explanation will be described hereinafter.

When the operation switch (not illustrated) of the facial care apparatus 100 is turned on, the blower 130 and the ion generation device 180 are operated. The external air is sucked into the facial care apparatus 100, the sucked air is fed to the ion generation device 180 via the filter 160 and the wind directing body 170. The air enhanced in wind velocity by the wind directing body 170 passes through the vicinity of the ion generation device 180 to thereby become the air containing many ions, and the air containing the ions is compressed and blown from the blowoff port 150 with the port diameter made smaller than the other diameters of the casing. The ions shown by the positive ions (for example, the white circles) and the negative ions (for example, the black circles) generated by the ion generation device 180 are carried by the air current which is turned and flows in to head for the blowoff port 150. The air is compressed in the narrow blowoff port 150, and the air which contains the ions with high density is released to the ion compressed region 20 shown by the dashed line. Because the air which includes the ions and flows down is in the turned state by the blade pieces 175, the air keeps the compressed state, and does not expand and is not reduced in the release speed in the ion compressed region 20.

The ions contained in the air which is supplied from the facial care apparatus 100 have the compositions of the positive ions H₃O⁺(H₂O)m (m is 0 or an optional natural number) and the negative ions O₂⁻(H₂O)n (n is 0 or an optional natural number) as described in embodiment 1. It is conceivable that the positive ions and the negative ions of a high concentration having the compositions intensively hit against a face (skin), whereby nano-size water molecules are generated on the skin surface as expressed by the following chemical formulas described above, and the water molecules are effectively adsorbed by the skin.

(1) H₃O⁺ + O₂⁻ → ·OH + H₂O₂

(2) H₃O⁺ + O₂⁻ → ·O₂H + H₂O

(3) 2H₂O₂ → 2H₂O + O₂

The facial care apparatus 100 of the present invention is of a method which uses water generated by the chemical reactions as described above, and does not directly spray water. Therefore, the velocity of the air current hitting a skin and the concentration of ions are important. Further, if the air current (air blow) is too fast, the skin is dried due to the air current.

Unlike an ordinary hair drier and air-conditioner such as an air cleaner, in the facial care apparatus of the present invention the face of a user in close proximity is the target, and therefore, the air current which is let off is required to be strictly "gentle". A distance L from an assumed target (face of a user) is around 30 cm. The velocity of the wind hitting the face is required to be approximately 1 m at the maximum. Therefore, it is necessary to blow air while detecting the velocity of the air current to be fed.

Thus, generation of ions and the wind velocity will be described.

The number of ions which can be fed out of the ion generation device 180 is significantly influenced by the velocity of the wind which passes through the vicinity of the discharge electrodes. More specifically, as the wind velocity is higher, the number of ions which are released increases more. Accordingly, the wind velocity needs to be increased in the vicinity of the ion generation device 180. Meanwhile, the upper limit of the velocity of the air which is released from the blowoff port 150 is approximately 1 m at the utmost as described above. Because the casing 120 of the facial care apparatus 100 cannot be configured to be thin for the reason of housing the blower 130 and the ion generation device, the wind velocity in the region of the ion generation device 180 is enhanced by contracting the air current by the wind directing body 170, and ions are efficiently transferred.

As above, in the facial care apparatus 100, the passage gap 173 of the wind directing body 170 is narrowed to increase the wind velocity in the vicinity of the ion generation device 180 to increase generation of ions, and the sectional area of the blowoff port 150 is made larger than the passage gap 173 to reduce the velocity of the wind which is blown from the blowoff port 150, while in order to cause the air current which is blown from the blowoff port 150 and contains positive and negative ions to reach a distant place, the air current is turned with the blade pieces 175 of the wind directing body 170 and the wind velocity of the blowoff port 150 is kept.

### [Embodiment 4]

An embodiment includes an ion detector 200 in addition to the configuration of the beauty apparatus described in embodiment 3. The ion detector 200 is controlled to detect ions released by the control unit not illustrated if there is ion release of a predetermined value or more.

The air which is released from the blowoff port 150 has the number of ions contained therein abruptly decreased as the distance becomes farther. Further, the number of ions is influenced by the wind blowing velocity, and therefore, the wind blowing velocity is determined by an experiment. In the embodiment, the ion generation device and the wind velocity are regulated so that the number of ions is 7,000/cm³ on a human face surface.

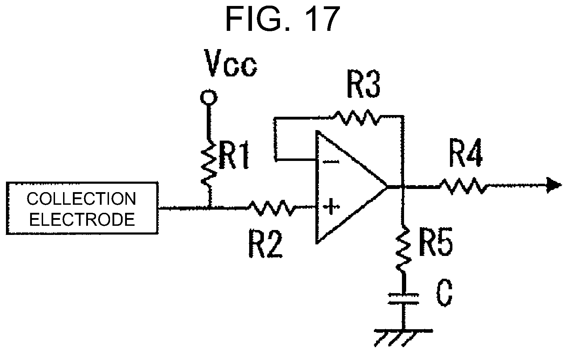

The number of ions released from the ion generation device is also influenced by the frequency of a high voltage which is applied to the discharge electrodes, besides being significantly influenced by the wind velocity as described above. In general, as the frequency is higher, the number of ions which are released increases more. The number of ions which are generated differs according to a discharge type and an electrode shape, and therefore, the conditions are set in the device which is decided to be used in advance. A drive circuit of the ion detector as a one example of the mode of carrying out the invention is shown in FIG. 17. In the circuit, R1 is set as 1 GΩ, R2 to R5 are set as 10 kΩ, C is set as 1000 pF and Vcc is set as 5 V.

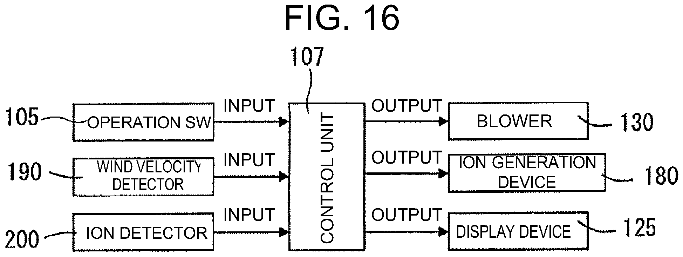

Next, control of the facial care apparatus 100 will be described according to FIG. 16.

When the operation switch 105 is inputted, control signals are outputted to the blower 130, the ion generator 180, and a display device 125 from a control unit 107. The control unit 107 is configured to output control signals to the blower 130 and the ion generation device 180 and control the wind velocity/ion concentration and the like of the air released from the facial care apparatus 100, when the wind velocity detector 190 detects the wind velocity which is a set wind velocity or more or the set wind velocity or less, and when the ion detector 200 detects an ion concentration of a value other than the set value.

### [Industrial Applicability]

By installing the facial care apparatus of the present invention at bedding, a skin is not dried during sleep, and a beauty effect can be obtained. Suspended bacteria can be removed by ions, and therefore, the environment with purified air can be supplied.

### [Reference Signs List]

1 voltage application needle electrode (positive ion generation electrode)
2 voltage application needle electrode (negative ion generation electrode)
3 ground (counter) electrode
4 high-voltage generation device
5 high-voltage generation device
6 ion generation apparatus
7 blowing fan
20 ion compressed region
60 ion generation element
100 facial care apparatus
130 fan
140 suction port
150 blowoff port
160 filter
170 wind directing body
180 ion generation device
190 wind velocity detector
200 ion detector

## Claims

1. A method for increasing a moisture content of a skin surface and improving a moisture retaining function of a dermis by a beauty apparatus comprising a blower, a case having a blow off port (150) and an ion generating device, **characterized in that**:
irradiating a skin surface simultaneously with positive ions and negative ions which are generated by the ion generating device from an air by an electric discharge in an atmosphere,
wherein concentrations of the positive and negative ions for irradiating the skin surface are of 7000/cm³ or more for the respective positive and negative ions.

2. The method according to claim 1,
wherein the positive ions are H₃O⁺(H₂O)m, wherein m=0 or an optional natural number, and the negative ions are O₂⁻(H₂O)n, wherein n=0 or an optional natural number.

3. A beauty apparatus (100) for increasing moisture content of a skin surface and improving a moisture retaining function of a dermis, the beauty apparatus comprising:
a blower (130);
a case (120) containing said blower and having a blow off port (150); and
an ion generating device (180) downstream of the blower in the case and configured to generate positive ions and negative ions from an air within the case (120) by an electric discharge in an atmosphere; **characterized by** comprising
a control unit (107) adapted to control the ion generating device (180) to irradiate a skin surface simultaneously with the positive and negative ions, emitted through the blow off port (150), each at a concentration of 7000/cm³ or more.

4. The beauty apparatus (100) according to claim 3, wherein the ion generating device is configured to generate H₃O⁺(H₂O)m, wherein m is 0 or an optional natural number, as the positive ions, and
O₂⁻(H₂O)n, wherein n is 0 or an optional natural number, as the negative ions.

## Patentansprüche

1. Verfahren zur Erhöhung eines Feuchtigkeitsgehalts einer Hautoberfläche und Verbesserung einer feuchtigkeitsbindenden Funktion einer Dermis durch eine Schönheitsvorrichtung, die Folgendes umfasst: ein Gebläse, ein Gehäuse, das eine Ausblasöffnung (150) aufweist, und eine ionenerzeugende Vorrichtung,
**dadurch gekennzeichnet, dass**:
Bestrahlen einer Hautoberfläche gleichzeitig mit positiven Ionen und negativen Ionen, die durch die ionenerzeugende Vorrichtung aus einer Luft durch eine elektrische Entladung in einer Atmosphäre erzeugt werden,
wobei Konzentrationen der positiven und negativen Ionen für das Bestrahlen der Hautoberfläche 7000/cm³ oder mehr für die jeweiligen positiven und negativen Ionen betragen.

2. Verfahren nach Anspruch 1,
wobei die positiven Ionen H₃O⁺(H₂O)m sind, wobei m = 0 oder eine optionale natürliche Zahl ist, und die negativen Ionen O₂⁻(H₂O)n sind, wobei n = 0 oder eine optionale natürliche Zahl ist.

3. Schönheitsvorrichtung (100) zur Erhöhung eines Feuchtigkeitsgehalts einer Hautoberfläche und Verbesserung einer feuchtigkeitsbindenden Funktion einer Dermis, wobei die Schönheitsvorrichtung Folgendes umfasst:
ein Gebläse (130);
ein Gehäuse (120), das das Gebläse enthält und eine Ausblasöffnung (150) aufweist; und
eine ionenerzeugende Vorrichtung (180), die dem Gebläse in dem Gehäuse nachgelagert ist und zur Erzeugung von positiven Ionen und negative Ionen aus einer Luft innerhalb des Gehäuses (120) durch eine elektrische Entladung in einer Atmosphäre konfiguriert ist; gekennzeichnet, dass sie Folgendes umfasst:
eine Steuereinheit (107), die zur Steuerung der ionenerzeugenden Vorrichtung (180) geeignet ist, um eine Hautoberfläche gleichzeitig mit den positiven und negativen Ionen zu bestrahlen, die durch die Ausblasöffnung (150) jeweils in einer Konzentration von 7000/cm³ oder mehr abgegeben werden.

4. Schönheitsvorrichtung (100) nach Anspruch 3, wobei die ionenerzeugende Vorrichtung zur Erzeugung von Folgenden konfiguriert ist: H₃O⁺(H₂O)m, wobei m 0 oder eine optionale natürliche Zahl ist, als positive Ionen und
O₂⁻(H₂O)n, wobei n 0 oder eine optionale natürliche Zahl ist, als negative Ionen.

## Revendications

1. Procédé permettant d'augmenter une teneur en eau d'une surface de peau et d'améliorer une fonction de rétention d'eau d'un derme au moyen d'un appareil de beauté comprenant une souffleuse, un étui ayant un orifice d'évacuation (150) et un dispositif de génération d'ions, **caractérisé en ce que** :
l'irradiation simultanée d'une surface de peau avec des ions positifs et des ions négatifs qui sont générés au moyen du dispositif de génération d'ions à partir un air au moyen d'une décharge électrique dans une atmosphère,
dans lequel les concentrations des ions positifs et négatifs destinés à l'irradiation de la surface de peau sont supérieures ou égales à 7000/cm³ pour les ions positifs et négatifs respectifs.

2. Procédé selon la revendication 1,
dans lequel les ions positifs sont H₃O⁺(H₂O)m, dans lequel m = 0 ou un nombre naturel éventuel, et les ions négatifs sont O₂⁻(H₂O)n, dans lequel n = 0 ou un nombre naturel éventuel.

3. Appareil de beauté (100) permettant d'augmenter la teneur en eau d'une surface d'eau et d'améliorer une fonction de rétention d'eau d'un derme, l'appareil de beauté comprenant :
une souffleuse (130),
un étui (120) contenant le ladite souffleuse et ayant un orifice d'évacuation (150) ; et
un dispositif de génération d'ions (180) situé en aval de la souffleuse dans l'étui et conçu pour générer des ions positifs et des ions négatifs à partir d'un air à l'intérieur de l'étui (120) au moyen d'une décharge électrique dans une atmosphère ; **caractérisé en ce qu'**il comprend
une unité de commande (107) conçue pour commander le dispositif de génération d'ions (180) afin d'irradier simultanément une surface de peau avec les ions positifs et négatifs, émis à travers l'orifice d'évacuation (150) chacun à une concentration supérieure ou égale à 7000/cm³.

4. Appareil de beauté (100) selon la revendication 3, dans lequel le dispositif de génération d'ions est conçu pour générer H₃O⁺(H₂O)m, dans lequel m = 0 ou un nombre naturel éventuel en tant qu'ions positifs, et
O₂⁻(H₂O)n, dans lequel n = 0 ou un nombre naturel éventuel, en tant qu'ions négatifs.
